(19) Europäisches Patentamt
European Patent Office
Office européen des brevets

(11) **EP 2 105 107 A1**

(12) **EUROPÄISCHE PATENTANMELDUNG**

(43) Veröffentlichungstag:
**30.09.2009 Patentblatt 2009/40**

(51) Int Cl.:
***A61B 19/00*** *(2006.01)*

(21) Anmeldenummer: **08153407.5**

(22) Anmeldetag: **27.03.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **BrainLAB AG**
**85622 Feldkirchen (DE)**

(72) Erfinder:
• **Manus, Johannes**
**81479, München (DE)**

• **Stumpf, Tanja**
**81543, München (DE)**
• **Fleig, Oliver**
**85598 Baldham (DE)**
• **Haberl, Martin**
**81373 München (DE)**
• **Haimerl, Martin**
**82205 Gilching (DE)**

(74) Vertreter: **Schwabe - Sandmair - Marx**
**Patentanwälte**
**Stuntzstrasse 16**
**81677 München (DE)**

(54) **Kalibrierungsverfahren für achsenbestimmte medizinische oder medizintechnische Instrumente**

(57)     Die Erfindung betrifft ein Kalibrierungsverfahren für ein achsenbestimmtes medizinisches oder medizintechnisches Instrument, bei dem
- das Instrument (1, 10), das sich im Ortungsbereich eines medizintechnischen Trackingsystems (20) befindet, derart positioniert wird, dass es sich um seine ortsfeste oder ortsbestimmte Achse (2, 12) drehen kann;
- das Instrument (1, 10) um die Achse (2, 12) gedreht wird, wobei eine Referenz (5, 15), die sich am Instrument (1, 10) befindet oder ortsfest gegenüber dem Instrument angeordnet bzw. an ihm befestigt ist, an mindestens zwei Positionen zu liegen kommt;
- die mindestens zwei Positionen räumlich mit Hilfe des Trackingsystems (20) bestimmt werden;
- aus den bestimmten Positionen die Achse (2, 12) des Instruments (1, 10) räumlich bestimmt wird; und bei dem
- die so bestimmte Achse (2, 12) dem getrackten Instrument (1, 10) zugeordnet wird.

Fig. 1

**Beschreibung**

Die Erfindung betrifft ein Kalibrierungsverfahren für ein achsenbestimmtes medizinisches oder medizintechnisches Instrument. Speziell ist die Erfindung im Bereich der medizintechnischen Instrumentennavigation angesiedelt, und sie befasst sich damit, die räumliche Lage der Achse eines achsenbestimmten Instruments festzustellen sie und diesem Instrument zuzuordnen, um mit dieser Information die weitere Navigation des Instruments zu unterstützen. Diesen Vorgang nennt man "Kalibrierung" des Instruments.

Die angesprochenen "aahsenbestimmten Instrumente" sind Instrumente, die entweder ihrer Gestalt nach oder ihrer Funktion nach länglich aufgebaut sind und bei deren Einsatz es darauf ankommt, wo die Achse oder Achsenfunktionslinie des Instruments im Raum und relativ zu anderen navigierten Objekten (andere Instrumente, Patientenkörperteile, ...) liegt, Beispiele für solche achsenbestimmten Instrumente sind Zeigegeräte bzw. Pointer, die entlang ihrer Achse Trajektorien anzeigen oder Reiben - bzw. Bohrer und deren Führungen, die eine Bearbeitungsrichtung entlang ihrer Achse angeben

Die Kalibrierung wird im Stand der Technik ganz allgemein mit Hilfe von Kalibrierungsinstrumenten durchgeführt. Solche Kalibrierungsinstrumente besitzen Anlageflächen, Anlagekanten oder Punktvertiefungen, sie sind ihrer Form nach im Navigationssystem genau bekannt und beschrieben und werden selbst räumlich lokalisiert, also mit Hilfe eines Trackingsystems getrackt, das einem Navigationssystem zugeordnet ist. Wenn dann ein medizinisches Instrument an ein solches Kalibrierungsinstrument angelegt oder dort ausgerichtet wird, kann - weil die räumliche Position des Kalibrierungsinstruments durch dessen Trackingreferenz bekannt ist - auch die räumliche Lage einer geometrischen Eigenschaft, also beispielsweise eine Achsenlage des Instruments bestimmt werden. Man hilft sich also bei dieser Instrumentenkalibrierung mit einem getrackten Kalibrierungsinstrument.

Kalibrierungssysteme, die Kalibrierungsinstrumente verwenden sind beispielsweise aus der CA 2440872 A1, der EP 0 904 735 A2 und der WO 02/061371 A1 bekannt.

Ein Nachteil dieser bekannten Kalibrierungssysteme liegt gerade darin, dass ein solches Kalibrierungsinstrument zur Verfügung gestellt werden muss. Getrackte Kalibrierungsinstrumente sind relativ teuer in der Herstellung, weil sie mit sehr geringen Toleranzen gearbeitet sein müssen. Ferner sind sie relativ anfällig, denn die Position der Trackingreferenz am Kalibrierungsinstruments muss immer dieselbe sein. Wenn das Instrument einmal herunterfällt und dabei auch nur eine geringe relative Verschiebung der Trackingreferenz stattfindet, ist es unbrauchbar. Ferner beansprucht das separate Tracking des Kalibrierungsinstruments Resourcen.

Es ist die Aufgabe der vorliegenden Erfindung, ein Kalibrierungsverfahren für achsenbestimmte medizinische oder medizintechnische Instrumente bereitzustellen, welches optimiert und insbesondere unaufwendig ist. Diese Aufgabe wird durch ein Kalibrierungsverfahren gemäß dem Anspruch 1 gelöst Die Unteransprüche definieren bevorzugte Ausführungsformen der Erfindung.

Das erfindungsgemäße Kalibrierungsverfahren weist die folgenden Schritte auf;

- das Instrument (1, 10), das sich im Ortungsbereich eines medizintechnischen Trackingsystems (20) befindet, wird derart positioniert, dass es sich um seine ortsbestimmte Achse (2, 12) drehen kann;
- das Instrument (1, 10) wird um die Achse (2, 12) gedreht, wobei eine Referenz (5, 15), die sich am Instrument (1, 10) befindet oder ortsfest gegenüber dem Instrument angeordnet bzw. an ihm befestigt ist, an mindestens zwei Positionen zu liegen kommt;
- die mindestens zwei Positionen werden räumlich mit Hilfe des Trackingsystems (20) bestimmt;
- aus den bestimmten Positionen wird die Achse (2, 12) des Instruments (1, 10) räumlich bestimmt; und
- die so bestimmte Achse (2, 12) wird dem getrackten Instrument (1, 10) zugeordnet.

Die Erfindung macht also gerade von den Rotationssymmetrie-Merkmalen bestimmter Instrumente Gebrauch, und sie nutzt diese Merkmale zur Umsetzung in einem einfachen Kalibrierungsverfahren. Mit anderen Worten wird die Lage der Instrumentenachse bestimmt, indem man das Instrument gerade um diese Achse dreht und einen bestimmten Punkt auf dem Instrument bei dieser Drehbewegung positionell an mindestens zwei Stellen bestimmt. Man muss lediglich dafür sorgen, dass die Achse ortsbestimmt oder ortsfest ist. Die vorliegende Erfindung hat also realisiert, dass sich gerade die drehsymmetrische Eigenschaft eines achsenbestimmten Instruments zu seiner Kalibrierung nutzen lässt. Man muss lediglich für eine ausreichende Positionstreue bzw. -bestimmbarkeit während der Kalibrierungs-Drehung sorgen, und hierfür ist kein getracktes Kalibrierungsinstrument notwendig.

Die Kalibrierung wird also einfacher, kostengünstiger und unaufwändiger.

Bei einer Ausführungsform des erfindungsgemäßen Verfahrens wird die Zuordnung zwischen Achse und Instrument als Instrumenten-Kalibrierungsinformation einem medizintechnischen Navigationssystem zur Verfügung gestellt, das mit dem Trackingsystem verknüpft ist Ist das Instrument einmal kalibriert, kann es im weiteren Verlauf der Operation immer korrekt navigiert werden.

Die Achse kann erfindungsgemäß in unterschiedlicher Weise bestimmt werden Eine Möglichkeit ist die Verwendung einer Eigenwertanalyse der Bewegung der Referenz an mindestens zwei Positionen. Auch kann die von der Referenz beschriebene Bahn (bei der Drehung) eine Kreisbahn sein oder ein Teil davon, und die Achse wird dann als Normale zur Kreisflächenebene durch den Kreismittelpunkt bestimmt. Zu beiden Verfahrensarten wird später noch detaillierter Stellung genommen.

Als Trackingsystem kann ein beliebiges Trackingsystem verwendet werden, und Beispiel hierfür wären ein optisch aktives oder passives, ein magnetisches, ein Radio-Frequenz- oder ein Ultraschall-Trackingsystem.

Die Referenz kann bei einer Variante der Erfindung direkt durch das Trackingsystem lokalisiert werden, insbesondere kann sie ein dem Trackingsystem zugeordneter Referenzmarker sein, der am Instrument befestigt ist. Die Drehung um die Achse wird dann eine Kreisbahn oder Teil-Kreisbahn des Referenzmarkers erzeugen.

Eine andere Möglichkeit besteht in der indirekten Lokalisierung der Referenz durch das Trackingsystem, und dies ist beispielsweise möglich, wenn diese Referenz während der Kreisbewegung mit einem getrackten Zeigegerät oder Pointer angefahren wird (die Pointerspitze bleibt bei der Bewegung auf der Referenz). In diesem Falle ist es von Vorteil, wenn die Referenz ein unfehlbar anfahrbarer Referenzpunkt am Instrument ist, insbesondere eine Vertiefung am Instrument oder eine Aussparung, welche die Spitze eines Pointers aufnehmen und festhalten kann

Gemäß einer Ausführungsform der vorliegenden Erfindung wird das Instrument durch eine ungetrackte Kalibrierungsstütze gehaltert, wenn es derart positioniert ist, dass es sich um seine ortsfeste Achse drehen kann. Ein solcher Kalibrierungshalter kann speziell eine zylindrische Aufnahme für den die Achse umgreifenden Schaft des Instruments aufweisen.

Die Erfindung umfasst ferner ein Programm, das, wenn es auf einem Computer läuft oder in einem Computer geladen ist, den Computer veranlasst, ein Verfahren durchzuführen, wie es oben in verschiedenen Ausführungsformen beschrieben wurde. Sie umfasst auch ein Computerprogramm-Speichermedium mit einem solchen Programm.

Die Erfindung wird im Weiteren anhand der beiliegenden Zeichnungen und der Beschreibung von Ausführungsformen näher erläutert Sie kann hierin beschriebenen Merkmale einzeln sowie in jedweder sinnvollen Kombination umfassen, und sie richtet sich insbesondere auch auf die vorrichtungsgemäße Umsetzung - auch wenn dies nicht in jedem Fall ausdrücklich erwähnt wird In den beiliegenden Zeichnungen zeigen:

Fig. 1 ein medizinisches Instrument, das erfindungsgemäß kalibriert werden kann;

Fig. 2 ein Beispiel für eine erfindungsgemäße Instrumentenkalibrierung mit einem Kalibrierungshalter;

Fig. 3 eine Ausführungsform eines erfindungsgemäßen Kalibrierungs- verfahrens bei einem Bohrwerkzeug, und

Fig. 4 ein Ablaufdiagramm für eine Ausführungsform der vorliegenden Erfindung.

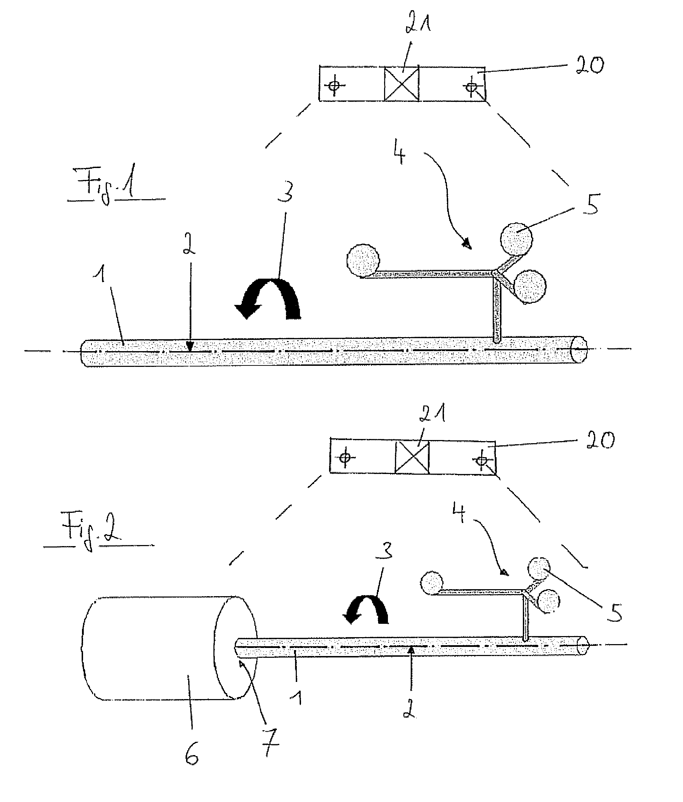

Die Figuren 1 und 2 verdeutlichen eine erste Ausführungsform der vorliegenden Erfindung, welche eine Kalibrierung zur Bestimmung der Achse eines medizinischen Instruments, nämlich eines Pointers 1, betrifft, der in einem Navigationssystem verwendet wird. Das Navigationssystem und ein zugehöriges Trackingsystem sind in den Figuren 1 bis 3 jeweils nur schematisch angedeutet; das Navigationssystem hat das Bezugszeichen 21, das Trackingsystem (zwei Kameras mit gestricheltem Blickfeld) das Bezugszeichen 20 erhalten.

Kalibriert werden soll nach einer ersten Ausführungsform der vorliegenden Erfindung, das in den Figuren 1 und 2 dargestellte Instrument 1, das vereinfacht einen Pointer darstellt, dessen Schaft die Achse 2 aufweist. Am Pointer ist ein Referenzstern 4 mit drei Reflektionsmarkern für das Trackingsystem 20 angebracht, und einer der Marker wird im Weiteren als Referenz 5 bezeichnet. Der Pfeil 3 zeigt in beiden Figuren eine Bewegung um die Achse 2 an, und diese Bewegung wird eine Drehbewegung mit ortsfester Achse sein, wenn - wie in Figur 2 dargestellt - der Pointer 1 in eine zylindrische Innenaufnahme 7 einer Kalibrierungsstütze 6 eingebracht wird, der starr befestigt ist (im Koordinatensystems des Trackingsystems 20). Die Aufnahme 7 der Kalibrierungsstütze 6 ist nun mit soviel Spiel behaftet, dass der Pointer 1 gedreht werden kann, und der Pointer stößt an seinem vorderen Ende auch an einem hier nicht sichtbaren Anschlag an. Die Kalibrierungsstütze 6 muss nicht trackbar sein, seine Position ist innerhalb des Blickfeldes des Trackingsystems 20 beliebig.

Die Achse muss während der Drehung des Instruments nicht unbedingt ortsfest sein, sondern lediglich ortsbestimmt. Eine ortsfeste Achse fällt unter diesen Begriff, aber auch eine Achse, die relativ zu einer anderen Vorrichtung ortsfest ist, welche von dem Trackingsystem getrackt werden kann.

Das Instrument kann entweder kontinuierlich gedreht werden, während die Positionen der Referenz 5 aufgezeichnet werden, oder mehrere Einzelpositionen der Referenz 5 während der Drehbewegung (mindestens zwei) werden aufgezeichnet. Die Achse kann dann aus diesen aufgezeichneten Positionen bzw. aus der aufgezeichneten Bewegung durch eine Eigenwertanalyse der Instrumentenbewegung errechnet werden, wobei die Eigenwerte der Bewegung der Rotationsachse des Instruments entsprechen. Eine solche Eigenwertanalyse findet wie folgt statt:

Die Eigenwerte $x_i$ werden aus der Bewegungsmatrix einer Referenz berechnet, wobei die Bewegungsmatrix den Übergang von Position $P_x$ zu $P_{x+1}$ darstellt.

1.) Berechnung der Bewegungsmatrix $T$:

$$T = M_x * M_{x+1}^{-1},$$

wobei $M_x$ die Transformation von Kamera zu Referenzgeometrie zum Zeitpunkt x und $M_{x+1}$ die Transformation von Kamera zu Referenzgeometrie zum Zeitpunkt x+1 sind

2.) Berechnung der Eigenwerte $\lambda_i$:

$$\det(T - \lambda E) = 0$$

wobei E die Einheitsmatrix ist

3.) Berechnung der Eigenvektoren $x_i$ durch:

$$(T - \lambda_i E)x_i = 0$$

Mit den Kenntnissen der Eigenwerte der Bewegung und damit der Rotationsachse lässt sich diese dem Instrument zuordnen und die Kalibrierung ist durchgeführt.

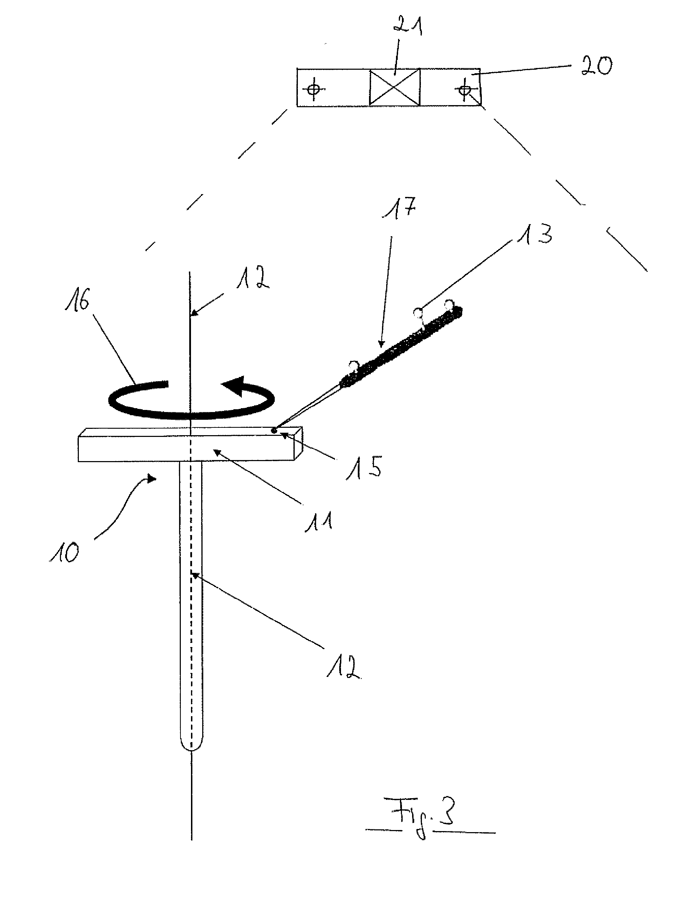

Eine weitere Möglichkeit der Durchführung eines erfindungsgemäßen Kalibrierungsverfahrens wird im Weiteren anhand der Figur 3 dargestellt. Die Figur 3 zeigt wieder einen navigierten, getrackten Bereich mit einem Instrument 10, nämlich einem Bohrwerkzeug, das einen Handgriff 11 aufweist. Die Achse des Bohrwerkzeugs 10 ist mit dem Bezugszeichen 12 angedeutet und die Drehung um diese Achse mit dem Bezugszeichen 16 Der Griff 11 weist eine Einkerbung 15 für die Spitze eines Pointers 17 auf, der mit Referenzreflektoren, von denen einer das Bezugszeichen 13 erhalten hat, im Trackingsystem 20 getrackt wird.

Bei dieser Ausführungsform wird aufgezeigt, wie erfindungsgemäß auf vorkalibrierte Werkzeuge oder getrackte Kalibrierungsinstrumente verzichtet werden kann und eine Kalibrierung lediglich mit einem Standard-Pointer und einem festen Punkt (Referenz) durchgeführt werden kann.

Gemäß der Ausführungsform der Figur 3 bestimmt das erfindungsgemäße Verfahren die Position einer Achse im dreidimensionalen Raum, z.B. bei der Schulterchirurgie die Achse des Implarttatkanals (Oberarmknochen). Die Achse kann nach dem Bohren ermittelt werden, wenn das Bohrwerkzeug (Reibwerkzeug) noch im aufgebohrten Kanal vorhanden ist..

Der Kanal für das Oberarmknochen-Implantat wird manuell unter Verwendung des Bohr- oder Reibwerkzeuges 10 mit dem T-förmigen Griff 11 gebohrt bzw. gefräst. Der Griff 11 ist am Bohrwerkzeug mit Hilfe eines Ratschenmechanismus befestigt, d.h. er kann in der Richtung entgegen der Bohrdrehung sehr leicht gedreht werden. Der Pointer 17, dessen Position über die Markeranordnung (Marker 13 und die beiden anderen Marker) vom Trackingsystem 20 getrackt wird, wird an einem der Arme des Griffes 11 so platziert, dass die Spitze in eine Vertiefung 15 eindringt, aus der sie nicht ausrutschen kann. Durch die Markeranordnung mit den Markern 13 wird die Spitze des Pointers 17 trackbar, und damit auch der Punkt 15, der in diesem Fall die Referenz ist und den die Pointerspitze nicht verlässt.

Zur Kalibrierung der Achse 12 des Instruments 10 wird nun der Griff 11 mit der Pointerspitze in der Vertiefung 15 in der leichten Drehrichtung der Ratsche gedreht. Der Punkt 15 kann dabei durch das Trackingsystem getrackt werden, weil der Pointer 17 getrackt wird, dessen Spitze eine Kreisbewegung ausführt.

Wenn nun wiederum genügend Pointerspitzen-Positionen erfasst worden sind, um die Parameter des Kreises, also seine Raumlage zu bestimmen, kann auch die Normale durch den Kreismittelpunkt bestimmt werden, und diese Normale entspricht dann der Instrumentenachse 12. Auch hier wird wiederum die Kreisbewegung eines Referenzpunktes auf dem Instrument verwendet, um die Achse zu bestimmten - die Verfolgung des Referenzpunktes 15 geschieht aber nicht direkt sondern indirekt über die Spitze des getrackten Pointers 17. Grundsätzlich kann auch hier die Eigenwertmethode, wie oben dargestellt, angewendet werden.

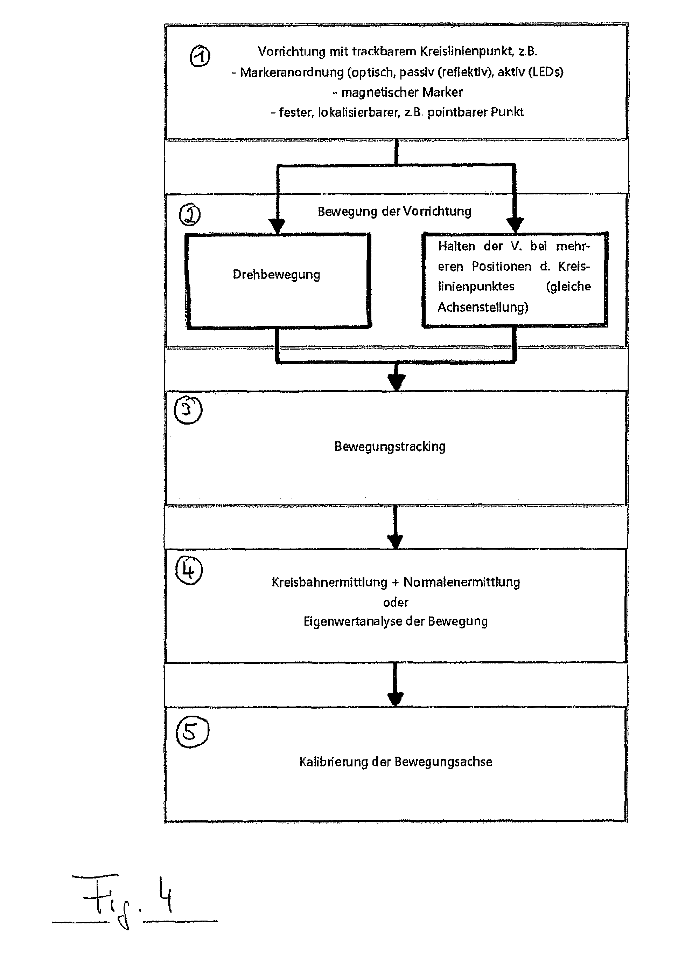

Zusammenfassend wird das erfindungsgemäße Verfahren nochmals in den Schritten 1 bis 5 im Ablaufdiagramm der Figur 4 dargestellt. Zuerst wird im Schritt 1 die zu kalibrierende Vorrichtung bereitgestellt, die einen trackbaren Kreislinienpunkt haben sollte, z. B. eine Markeranordnung (optisch, passiv (reflektiv), aktiv (LED's)), ein magnetischer Marker oder einen festen, lokalisierbaren, z. B. pointbaren Punkt. Danach erfolgt die Bewegung der Vorrichtung im Schritt 2, die eine Drehbewegung sein kann, aber auch aus einem Halten der Vorrichtung bei mehreren Dreh-Positionen des Kreislinienpunktes bestehen kann (gleiche Achsenstellung). Die Bewegung wird verfolgt durch das Bewegungstracking des Schrittes 3, so dass die Schritte 2 und 3 gleichzeitig oder zumindest überlappend geführt werden. Aus der getrackten Bewegung lässt sich nun gemäß einer Ausführungsform eine Kreisbahn und eine Normale durch den Kreismittelpunkt bestimmen (Achse), oder es wird unmittelbar eine Eigenwertanalyse der Bewegung durchgeführt, und beiden Prozesse resultieren in der Achsenbestimmung und damit in der Kalibrierung der Bewegungsachse des Instruments, indem die bestimmte Achse diesem Instrument zugeordnet wird.

**Patentansprüche**

1.   Kalibrierungsverfahren für ein achsenbestimmtes medizinisches oder medizintechnisches Instrument, bei dem

- das Instrument (1, 10), das sich im Ortungsbereich eines medizintechnischen Trackingsystems (20) befindet, derart positioniert wird, dass es sich um seine ortsfeste oder ortsbestimmte Achse (2, 12) drehen kann;
- das Instrument (1, 10) um die Achse (2, 12) gedreht wird, wobei eine Referenz (5, 15), die sich am Instrument (1, 10) befindet oder ortsfest gegenüber dem Instrument angeordnet bzw. an ihm befestigt ist, an mindestens zwei Positionen zu liegen kommt;
- die mindestens zwei Positionen räumlich mit Hilfe des Trackingsystems (20) bestimmt werden;
- aus den bestimmten Positionen die Achse (2, 12) des Instruments (1, 10) räumlich bestimmt wird; und bei dem
- die so bestimmte Achse (2, 12) dem getrackten Instrument (1, 10) zugeordnet wird.

2. Kalibrierungsverfahren nach Anspruch 1, bei dem die Zuordnung zwischen Achse (2, 12) und Instrument (1, 10) als Instrumenten-Kalibrierungsinformation einem medizintechnischen Navigationssystem (21) zur Verfügung gestellt wird, das mit dem Trackingsystem (20) verknüpft ist.

3. Kalibrierungsverfahren nach Anspruch 1 oder 2, bei dem die Achse (2) mit Hilfe einer Eigenwertanalyse der Bewegung der Referenz an mindestens zwei Positionen bestimmt wird.

4. Kalibrierungsverfahren nach einem der Ansprüche 1 bis 3, bei dem die die von der Referenz (5, 15) bei der Drehung beschriebene Bahn eine Kreisbahn oder ein Teil davon ist und die Achse (12) als Normale zur Kreisflächenebene durch den Kreismittelpunkt bestimmt wird.

5. Kalibrierungsverfahren nach einem der Ansprüche 1 bis 4, bei dem das Trackingsystem ein optisch aktives oder passives, ein magnetisches, ein Radio-Frequenz- oder ein Ultraschall-Trackingsystem ist.

6. Kalibrierungsverfahren nach einem der Ansprüche 1 bis 5, bei dem die Referenz (5) direkt durch das Trackingsystem (20) lokalisiert wird, insbesondere ein dem Trackingsystem (20) zugeordneter Referenzmarker (5) ist.

7. Kalibrierungsverfahren nach einem der Ansprüche 1 bis 5, bei dem die Referenz (15) indirekt durch das Trackingsystem (20) lokalisiert wird, insbesondere indem sie mit einem getrackten Zeigegerät oder Pointer (17) angefahren wird.

8. Kalibrierungsverfahren nach Anspruch 7, bei dem die Referenz ein unfehlbar anfahrbarer Referenzpunkt (15) am Instrument (10) ist, insbesondere eine Vertiefung oder Aussparung.

9. Kalibrierungsverfahren nach einem der Ansprüche 1 bis 7, bei dem das Instrument, wenn es derart positioniert wird, dass es sich um seine ortsfeste Achse (2, 12) drehen kann, durch eine ungetrackte Kalibrierungsstütze (6) gehaltert wird, insbesondere mit einer zylindrischen Aufnahme für den die Achse umgreifenden Schaft des Instruments.

10. Programm, das, wenn es auf einem Computer läuft oder in einem Computer geladen ist, den Computer veranlasst, ein Verfahren gemäß einem der Ansprüche 1 bis 9 durchzuführen.

11. Computerprogramm-Speichermedium, das ein Programm nach Anspruch 10 aufweist.

Fig.1

Fig.2

Fig. 3

Fig. 4

**Europäisches Patentamt**

## EUROPÄISCHER RECHERCHENBERICHT

Nummer der Anmeldung

EP 08 15 3407

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 02/061371 A (Z KAT INC [US]) 8. August 2002 (2002-08-08) * Seite 5, Zeile 30 - Seite 6, Zeile 1 * ----- | 11 | INV. A61B19/00 |
| X | WO 01/67979 A (ORTHOSOFT INC [CA]; JUTRAS SEBASTIEN [CA]; BROSSEAU ERIC [CA]; JANSEN) 20. September 2001 (2001-09-20) * Seite 1, Zeile 18 - Zeile 25 * ----- | 1-11 | |
| X | US 2006/094958 A1 (MARQUART JOEL G [US] ET AL) 4. Mai 2006 (2006-05-04) * Absatz [0007] * ----- | 1-11 | |
| A | DE 10 2006 026913 A1 (STRYKER LEIBINGER GMBH & CO KG [DE]) 12. April 2007 (2007-04-12) * Absätze [0049], [0056] * ----- | 1 | |

RECHERCHIERTE SACHGEBIETE (IPC)

A61B
G01B

Der vorliegende Recherchenbericht wurde für alle Patentansprüche erstellt

| Recherchenort | Abschlußdatum der Recherche | Prüfer |
|---|---|---|
| Den Haag | 25. September 2008 | Mayer-Martenson, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

**ANHANG ZUM EUROPÄISCHEN RECHERCHENBERICHT
ÜBER DIE EUROPÄISCHE PATENTANMELDUNG NR.**

EP 08 15 3407

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-09-2008

| Im Recherchenbericht angeführtes Patentdokument | Datum der Veröffentlichung | Mitglied(er) der Patentfamilie | | Datum der Veröffentlichung |
|---|---|---|---|---|
| WO 02061371 A | 08-08-2002 | CN | 1511249 A | 07-07-2004 |
| | | EP | 1364183 A1 | 26-11-2003 |
| | | JP | 2004529679 T | 30-09-2004 |
| WO 0167979 A | 20-09-2001 | AU | 4816101 A | 24-09-2001 |
| | | EP | 1265547 A1 | 18-12-2002 |
| | | JP | 2003534035 T | 18-11-2003 |
| | | US | 2003040879 A1 | 27-02-2003 |
| US 2006094958 A1 | 04-05-2006 | CA | 2585687 A1 | 08-06-2006 |
| | | EP | 1833400 A1 | 19-09-2007 |
| | | JP | 2008518688 T | 05-06-2008 |
| | | WO | 2006060107 A1 | 08-06-2006 |
| DE 102006026913 A1 | 12-04-2007 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CA 2440872 A1
- EP 0904735 A2
- WO 02061371 A1